(19) Europäisches Patentamt
European Patent Office
Office européen des brevets

(11) **EP 3 972 664 B1**

(12) **EUROPEAN PATENT SPECIFICATION**

(45) Date of publication and mention
of the grant of the patent:
**25.02.2026 Bulletin 2026/09**

(21) Application number: **20736395.3**

(22) Date of filing: **05.05.2020**

(51) International Patent Classification (IPC):
*A61M 1/16* *(2006.01)*    *A61M 1/36* *(2006.01)*
*A61M 60/113* *(2021.01)*    *A61M 60/117* *(2021.01)*
*A61M 60/232* *(2021.01)*    *A61M 60/279* *(2021.01)*
*A61M 60/38* *(2021.01)*    *A61M 60/515* *(2021.01)*
*A61M 60/523* *(2021.01)*

(52) Cooperative Patent Classification (CPC):
**A61M 1/1698; A61M 1/3666; A61M 60/113;**
**A61M 60/117; A61M 60/232; A61M 60/279;**
**A61M 60/38; A61M 60/515; A61M 60/523;**
A61M 2205/3334; A61M 2230/04; A61M 2230/42

(86) International application number:
**PCT/GB2020/051097**

(87) International publication number:
**WO 2020/234563 (26.11.2020 Gazette 2020/48)**

(54) **CONTROL SYSTEM**

STEUERUNGSSYSTEM

SYSTÈME DE COMMANDE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **21.05.2019 GB 201907166**

(43) Date of publication of application:
**30.03.2022 Bulletin 2022/13**

(73) Proprietor: **Haemair Limited**
**SA2 8PP (GB)**

(72) Inventors:
• **JOHNS, Richard William**
**Swansea University, Singleton Park Swansea**
**SA2**
**8PP (GB)**

• **KNIGHT, Ronald Kelvin**
**Swansea University, Singleton Park Swansea**
**SA2**
**8PP (GB)**

(74) Representative: **Murgitroyd & Company**
**165-169 Scotland Street**
**Glasgow G5 8PL (GB)**

(56) References cited:
**EP-A1- 2 004 255    WO-A1-2018/106164**
**WO-A1-92/00777    US-A- 5 695 717**
**US-A- 5 810 759**

EP 3 972 664 B1

**Description**

**Field of the Invention**

The present invention relates to control systems for extracorporeal life support (ECLS) apparatuses and similar apparatuses that support mammalian respiration and blood circulation.

**Background to the Invention**

There are many actual and proposed systems which attempt to control extracorporeal life support (ECLS) devices to provide physiologically healthy oxygen and carbon dioxide concentrations in the blood circulation.

These "healthy" concentrations are typically provided as set-points in a control system arranged to control the ECLS device. The set points may be selected from partial pressures or concentrations of oxygen and carbon dioxide, or oxygen saturation in the arterial blood, or corresponding measures in the venous blood. The controllers typically use measurements of selected variables to provide feedback control to maintain them at their set points.

Devices used with these automated systems typically measure oxygen and/or carbon dioxide concentration in the venous or arterial blood, oxygen and/or carbon dioxide partial pressure in the venous or arterial blood, and/or blood oxygen saturation in the venous or arterial blood, or a combination of some or all of these measurements. Under such circumstances, the autonomic nervous system can drive the respiratory system to potentially dangerous extreme values in a vain attempt to achieve its preferred blood gas concentrations. Similar considerations apply with "uncontrolled" ECLS in which the blood flow rate and gas flow rate through the oxygenator remain constant until manually adjusted by the operator. It is for such reasons that even the best automated systems known in the prior art require 24-hour human monitoring seven days a week. The human monitor can react to adjust set values (or directly adjust blood and gas flow rates) to prevent potentially dangerous conditions.

Current automated control systems process the measured values as input signals and produce outputs that control blood and gas flow rates, and gas composition through the ECLS device. For such systems, a clinician or perfusionist judges the target oxygen and carbon dioxide concentrations (and/or partial pressures/saturation levels) which are most desirable. These judged values then serve as set-points in the control system. These set-points are typically adjusted manually to maintain the patient's respiration within acceptable bounds. The setting of such values typically results from subjective judgements by the perfusionist or clinical staff as to the most desirable values for each measurand. It has been identified, however, that in practice the autonomic nervous system sets levels that differ from person to person. It may also set levels that change from time to time in response to changing levels of exertion or to acclimatization. As an example of person-to-person variation, venous oxygen saturations may range from 45% to 75% in a group of equally healthy people.

It is therefore desirable to provide a control mechanism for extracorporeal life support devices and systems which is responsive to the needs of an individual patient and objectively provides the most effective setting of an extracorporeal life support device for said patient. The control mechanism would preferably be portable, providing patients with improved mobility, while also overcoming the disadvantages of the currently available solutions.

EP2004255 describes a mass exchange apparatus for use in blood/air mass exchange comprising plural blood flow conduits for defining a blood flow from a blood flow inlet provided thereto; and plural air flow conduits for defining an air flow from an air flow inlet provided thereto. The plural air flow conduits and plural blood flow conduits at least partially comprise gas-permeable membrane material, and the conduits are arranged relative to each other such as to enable transfer of oxygen from the air flow to the blood flow and transfer of carbon dioxide from the blood flow to the air flow through the membrane material. The apparatus additionally comprises at least one sensor for sensing patient respiratory demand; and a controller for controlling the rate of blood/air mass exchange by separate control of the levels of carbon dioxide and oxygen in the air flow responsive to the sensing of patient respiratory demand by the sensor.

**Summary of the Invention**

Current devices take no account of the natural mammalian control system whereby the autonomic nervous system adjusts blood oxygen and carbon dioxide concentrations to match the metabolic demand. Without wishing to be bound by theory, the natural system provides feedback from measurements by chemoreceptors (particularly carbon dioxide measured by a carotid chemoreceptor) which are used to adjust heart rate and respiration rate. The autonomic nervous system also provides anticipatory control. Thus, it takes signals from other parts of the body (including eyes and ears, and a conscious intention to move) to provide increased oxygen in anticipation of a higher demand. In addition, the autonomic nervous system provides an "acclimatization" function, whereby its responses are changed depending on the environment (for example, lower oxygen concentration at higher altitude) and the state of the body (for example, reduced or increased lung efficiency). The autonomic nervous system expects that, when it increases blood circulation rate and ventilation (breathing) rate, the oxygen concentration in the blood will increase and the carbon dioxide concentration will decrease. Conversely, when it decreases blood circulation rate and ventilation rate, oxygen concentration will decrease and carbon dioxide concentration increase. It then increases or decreases these rates to achieve blood gas concentrations and partial pressures appropriate for the level of metabolic demand. In effect, the autonomic nervous system generates its own set points. These natural set points can differ significantly from person to person, and can depend on the level of exertion and the circumstances of the patient.

Devices that directly control blood oxygen and carbon dioxide concentrations through "set points" cannot replicate the subtle adaptability of the autonomic nervous system. Indeed, they can in some cases be in conflict with the system. For example, they can strive to achieve preferred blood gas concentrations that differ from those that would be set by the autonomic nervous system. The autonomic nervous system can then make large changes in heart rate in a vain attempt to achieve its own desired blood gas concentrations, partial pressures or saturations. Consequently, conventional "set point" control systems can result in unstable control requiring 24-hour human monitoring seven days a week. Similar considerations apply with "uncontrolled" ECLS systems in which the blood flow rate and gas flow rate through the oxygenator remain constant until manually adjusted by the operator. The human monitor can react to adjust set values (or directly adjust blood and gas flow rates) to prevent potentially dangerous conditions.

It is the objective of the current invention to provide a control system that integrates with the natural autonomic nervous system by using a surrogate of the nervous system output to control blood flow rate and gas transfer rates in an Extracorporeal Life Support System.

A further objective is to provide a control system for ventricular assist devices (VAD) that also integrates with the natural autonomic nervous system.

In view of the shortcomings of current control systems, a novel system is presented that integrates with, and closely mimics, the natural control system for a person with healthy lungs. It does not measure or directly control blood gas composition. Instead it uses a surrogate of the output from the autonomic nervous system. A preferred surrogate is heart rate. The autonomic nervous system tends to change heart rate and ventilation rate in synchronization. Thus, an increased demand for oxygen stimulates an increased ventilation rate to make more oxygen available in the lungs and an increased blood circulation rate to distribute the oxygen around the body. This synchronization also serves to transport the resulting increased carbon dioxide produced back to the lungs where the increased ventilation rate discharges the carbon dioxide to atmosphere. Where lung efficiency is reduced so that ECLS is needed, the novel control system aims to make the residual lung function, augmented by the ECLS system, to behave as a natural lung. Thus, with increasing heart rate (pulse rate or blood circulation rate) the ECLS system increases oxygen and carbon dioxide transfer so that the resulting transfer rates are similar to those from a healthy lung. Similarly, with decreasing oxygen demand, the heart rate slows and the ECLS system gives a lower gas transfer rate. It is optionally not essential that the combined lung plus ECLS exactly mimics any particular natural lung; the acclimatization capability of the autonomic nervous system can adjust its response (extent to which heart rate responds to changes in blood gas concentrations) to provide stable responses over a range of conditions. The resultant control requires only one robust non-invasive measure to control the ECLS. This robust, simple system preferably promises a more portable ECLS thus providing patients with improved mobility. It is well established that improved mobility improves recovery rates and shortens hospital stays. Improved ECLS control is an important step in furnishing ambulatory capability to patients and ultimately allowing such patients to live at home.

The novel control system recognizes that the response of an ECLS with a fixed transfer area differs from that of a natural lung in which blood flow rate and ventilation rate change concurrently with change in the effective transfer area. To enable the autonomic nervous system to control the ECLS, the control system must preferably control oxygen and carbon dioxide transfer rates to mimic those of a natural lung. There are 4 possible control variables:

- blood flow rate through an ECLS device;
- flow rate of a first gas through an ECLS device;
- flow rate of a second gas through an ECLS device; and/or
- effective area of an ECLS device.

In examples not according to the claimed invention, an alternative set of 4 control variables may be:

- blood flow rate through an ECLS device;
- flow rate of a gas from an oxygen concentrator through an ECLS device;
- oxygen concentration of a gas from an oxygen concentrator through an ECLS device; and/or
- effective area of an ECLS device.

For a venous/venous ECLS device, blood is taken from a vein (with low oxygen and high carbon dioxide concentration) leading to the heart and passed through an oxygenator. The oxygenated blood (with higher oxygen concentration and lower carbon dioxide concentration) is then preferably returned to a vein leading to the heart/lungs where any residual capacity in the lungs may complete the gas transfer, so that the gas concentrations in the blood leaving the heart/lungs are approximately the gas concentrations that would have resulted by passing through healthy lungs. In an arterial/arterial ECLS device, the ECLS preferably completes the oxygenation of the blood leaving the heart/lungs. Venous/arterial ECLS devices are not favoured because, in such devices, oxygenated blood bypasses the heart which, as a major muscle, has a significant oxygen demand. Similarly, arterial/venous ECLS devices are not favoured because oxygenated blood then bypasses part of the body, thus depriving that part of the body of oxygenated blood. A venous/venous device will be described, but those skilled in the art will recognize how the principles map directly into an arterial/arterial device. Blood flow rate can optionally be controlled within the limits of zero and a total flow rate through the vein from which the blood is taken. In practice, the minimum flow rate should be significantly above zero, for example above 50% of the minimal flow through the vein when the subject is at rest, or the residence time of the blood in the oxygenator will be so long that clots will form. Where less than the whole flow rate through the vein is taken, it is necessary to allow a margin below the whole flow rate because it is not desirable to have zero, or a very low flow through a vein immediately downstream of the extraction point. A low flow would result in a long residence time in that downstream length of vein with consequent risk of blood clots forming adjacent to, or immediately downstream of, the tapping point. For example, the maximum take-off rate could be at most 90% of the flow in the vein, so leaving a flow of at least 10% through the vein downstream. An exception to these limits may arise when the pressure drop through the device is sufficiently low for the whole flow to be taken through the device and returned to the vein at the take-off point. With sufficiently low pressure drop, no separate blood pump may be required.

Mixing two gas feeds, one providing a first gas having a high oxygen concentration and the other providing a second gas having a low oxygen concentration preferably allows the oxygen driving force to be adjusted by altering the proportions of the two gases.

Alternative embodiments are possible in which the gases are not fully premixed and/or flow unequally between the channels, and/or are controlled to flow unequally between the channels. The ECLS device may comprise an "effective area", the effective area describing the area of access for a gas to a gas permeable membrane, across which gas transfer is permitted into blood. The effective area of the device can optionally be altered, which may be by independently controlling access of gas flow rates to individual, or groups of, gas channels of the device. The gas flow through a number of channels can optionally be controlled, and the number may be reduced to zero to effectively remove the transfer area of part of the device.

It is necessary to adjust two of the above-described control variables, and to control gas concentrations of a first gas and a second gas (such as, for example, oxygen and air (comprising carbon dioxide) respectively) in the blood independently. In order to mimic a natural lung, both the oxygen and the carbon dioxide concentration in the resulting arterial blood are desired to match the concentrations that would arise from a healthy natural lung. These desired concentrations depend on an individual's metabolic demand. In order to achieve this match, the blood oxygen and blood carbon dioxide concentrations must be independently controlled whether or not the device is followed by a lung with a residual gas transfer capability. In the event of a fault, for example if one gas fails, it could be that carbon dioxide (if used alone as the second gas) would transfer to the blood. Such a transfer would likely kill a patient receiving treatment. It is preferred to remove carbon dioxide from the blood. Any carbon dioxide concentration in the second gas should therefore be low. The only reason that it might be preferable to have a concentration of carbon dioxide (a small concentration) in the second gas would be to prevent the carbon dioxide partial pressure in the blood of the patient from becoming too low. An alternative means of ensuring that carbon dioxide partial pressure is not too low is to make the total mixed gas flow rate through the ECLS device (in this example a membrane oxygenator) sufficiently low to ensure that carbon dioxide transfer from the blood causes a sufficient concentration of carbon dioxide in the mixed gas stream to limit further transfer of carbon dioxide from the blood. This latter means corresponds more closely to the natural control system which, of course, does not employ a carbon dioxide-containing gas. One preferred embodiment of the control system does not employ a prepared second gas containing carbon dioxide. One motivation for this is for mobility of a patient when receiving treatment - it is preferable for a patient not to be required to carry two gas bottles. Ideally, a single first gas cylinder would be employed along with an air pump (air being the second gas), the use of air providing a low carbon dioxide concentration while also reducing the necessity for a large volume oxygen cylinder. This option also opens the possibility of an alternative embodiment employing an oxygen concentrator. The same effect as adjusting the flow rates of a higher oxygen concentration stream (the first gas) and a lower oxygen concentration stream (the second gas) can therefore be achieved by controlling the oxygen concentration and flow rate from an oxygen concentrator.

In a preferable venous/venous embodiment, there are three manipulated variables:

- the blood flow rate through the device
- the flow rate of the first gas stream (rich in oxygen) into the device
- the flow rate of the second gas stream (having a lower concentration of oxygen) into the device

The two gas feeds are preferably mixed before entering the device, but may be mixed within the device.

The blood flow rate is set proportional to the blood flow through the vein from which it is taken. It is preferably set at a high proportion of the blood flow in the vein to ensure that, when remixed with the unoxygenated blood not passing through the device, the blood oxygenation remains high.

At maximum oxygen demand (as indicated by the blood flow rate) the first gas stream is set to its maximum flow rate and the second gas stream to a flow rate of zero.

At minimum oxygen demand (as indicated by blood flow rate) the first gas stream is set to a flow rate of zero. The second gas stream is adjusted to its maximum flow rate. This maximum flow rate is sufficiently low to ensure that carbon dioxide transferred from the blood to the flowing gas reaches a sufficiently high concentration to ensure that the autonomic nervous system maintains an adequate blood circulation rate. To those skilled in the art it will also be clear that similar controls can be achieved by employing oxygen concentrators instead of, or as well as, separate gas streams.

As an example, the blood and gas flows through the device could be controlled as follows:

$$f_D = kf \qquad\qquad (1)$$

$$g_1 = a + bf \qquad\qquad (2)$$

$$g_2 = c + df \qquad\qquad (3)$$

where k, a, b, c, d are constants, $f_D$ is the blood flow rate through the device, $g_1$ and $g_2$ are the flow rates of the first and second gas streams respectively, and "f" is the heart rate (pulse rate or blood flow rate from the heart). "k" is positive and selected to ensure that a large proportion of the blood flow through the vein is taken through the device. "b" is positive so that, at a maximum flowing blood flow rate, the first gas (oxygen) flow is a maximum. "a" is negative so that, at a minimum flowing blood flow rate, the first gas (oxygen) flow is zero. "c" is positive and "d" is negative so that at a maximum blood flow rate, the second gas (air) flow is zero.

There are many obvious variants of the above including, but not limited to:

- employing gas streams of differing compositions with the parameters in the equations suitably adjusted such that the oxygen transfer rate at minimum metabolic demand remains sufficient to support the resting oxygen demand;
- use of more than 2 gas streams;
- allowing any other algorithm, linear or non-linear that has a similar effect in maximizing gas transfer rates at high blood circulation and reducing transfer rates at lower blood circulation. Such algorithms may reduce the proportion of blood taken from the vein at low blood flow rates in order to reduce the amount of gas transfer to the main blood circulation;
- including a concentration of carbon dioxide in gas stream 2 so that, at the lowest blood flow rates, the autonomic nervous system still has an incentive to maintain the blood circulation;
- allowing unequal flow through parts of the ECLS mass exchanger (oxygenator) such that, in effect, the active mass transfer area can be increased or decreased; and
- any combination of the above variants.

To those skilled in the art it will be clear that similar control methodology can be applied not only to venous/venous systems but also to venous/arterial, arterial/venous and arterial/arterial systems

The common factor of all these control strategies is that all control actions are taken on the basis of a single surrogate measure of an output from the autonomic nervous system of the individual receiving ECLS. That measure may, itself, be made up of, or estimated from, one or more measurands, and may be a compound measurand. For example, a blood flow rate from a heart may be estimated from the product of a pulse rate and a stroke volume. A further common factor is that there is no feedback control other than that provided by the autonomic nervous system. Thus, as in equations (1) to (3) above, there is a direct relationship between the measured value and the controls; there are no error functions or differential equations determining control trajectories linking one level of metabolic demand to another. The trajectory is determined entirely by the autonomic nervous system. A preferable benefit of delegating control to the autonomic nervous system is that the anticipation and acclimatization features of the natural control system are incorporated in a way that is not possible with any system in which gas transfer rates are controlled manually or by automatic control systems that set out to control blood gas concentrations directly.

In accordance with a first aspect of the present invention, there is provided an extracorporeal life support device control system according to claim 1.

The extracorporeal life support device control system is preferably arranged to provide a blood flow rate within the extracorporeal life support device, the control system being further arranged to control, according to the measurand:

- a blood flow rate of blood flowing through the extracorporeal life support device.

The extracorporeal life support device control system is optionally arranged to control, according to the measurand:

- an effective area an extracorporeal life support device.

Preferably the extracorporeal life support device comprises a semipermeable membrane across which transfer of species through mass transfer may occur. Preferably the semipermeable membrane is exposed to flowing gas on one side and flowing blood on an opposing side, the exposed portion of the semipermeable membrane comprising the effective area. Preferably the control system of the present invention is arranged to adjust, according to the measurand, the effective area of the extracorporeal life support device.

In line with the above, the effective area, or active area, will be understood in the context of the present invention to mean an area of the extracorporeal life support device wherein gaseous transfer of species (such as during blood oxygenation) may occur. In an example, gaseous transfer may occur in one or more channels of the extracorporeal life support device. The control system in such an example may therefore, according to the measurand, prevent, limit, reduce, or otherwise alter access to the one or more channels in order to adjust the "effective area" of the extracorporeal life support device.

Embodiments will be appreciated wherein any of the first gas and/or the second gas may be comprised within a mixed phase treatment agent comprising both a gas and a liquid. Use of such a mixed-phase treatment agent may be suitable for simple simultaneous transfer of any combination of thermal energy (heat), gas and/or dissolved species to or from the blood of a patient receiving treatment. In such embodiments, the ECLS device is preferably a membrane oxygenator such as an extracorporeal membrane oxygenator (ECMO). In such embodiments, the measurand may be used to control the flow rate of the mixed-phase treatment agent, which may be controlled instead of the flow rate of the first gas.

The control system is preferably arranged to adjust a blood flow rate, and preferably arranged to adjust the proportions and flow rates of one or more gases in response to a measurand which is characteristic of a single autonomic nervous system output (such as, for example, heart rate) such that a resulting blood gas composition of blood flowing through the extracorporeal life support device approximates that which would arise from healthy lungs. It is recognized that heart rate is an example surrogate for an output from the autonomic nervous system that, in a healthy person, would control ventilation rate (frequency and depth of breathing). In this way, the autonomic nervous system may, for example, increase the heart rate in response to a higher metabolic demand, and the extracorporeal life support device consequently increases an oxygenation rate and carbon dioxide removal rate in response to the increased heart rate. Conversely, the autonomic nervous system may decrease the heart rate in response to a lower metabolic demand and the extracorporeal life support device (preferably a blood oxygenator) decreases gas transfer rates in response to the signal from the measure of heart rate.

Without wishing to be bound by theory, the autonomic nervous system detects changes in blood gas composition from chemoreceptors and changes the heart rate to pump an appropriate blood flow around the body. In a healthy subject, it also adjusts the ventilation rate to increase oxygen and carbon dioxide transfer in the lungs. In this way, the natural control system provides a stable response to changing metabolic demand. With the proposed ECLS control system, the system uses a measure of an autonomic nervous system output to adjust the gas transfer rate(s) in the ECLS device, preferably to mimic the behaviour of a healthy lung. In this way, the controlled ECLS device is integrated with the autonomic nervous system to give a stable response to changes in metabolic demand. This integration also furnishes features of the natural control system, such as anticipatory control and long-term acclimatization, not available to previous ECLS control systems.

The present invention is not intended to respond to direction of change, or rate or extent of change of the measurand, but instead detects an absolute measurand corresponding to an autonomic nervous system output (such as, for example, blood circulation rate; pulse rate; heart rate) at any given instant of time. The present system delegates response to such changes to the autonomic nervous system. The present invention does not use any feedback or set target levels. As such, unlike virtually all presently provided systems, the present control system does not deal with differential equations (rate of change) or integrals (extent of change integrated) over time.

The controller may preferably adjust one or more of: the blood flow rate; and the proportion of channels through which the gas or blood flows (the effective area). Preferably, the blood continues to flow equally through all channels. In order to control the effective mass transfer area in the device, the semipermeable membrane may preferably be divided into parallel sections. The effective area may preferably be reduced by reducing the gas flow through some channels to, at minimum effective area, reduce gas flow to zero in some channels. The gas flow rates are optionally adjusted in response to a measurement of heart rate such that the resulting blood gas compositions approximate those that would arise from healthy lungs.

In preferable embodiments the extracorporeal life support device comprises a pump arranged to be controlled to achieve a desired blood flow rate through the extracorporeal life support device.

The blood pump may take blood from a vein and send said blood through the life support device and return it to a vein. A wide variety of pump types are equally applicable for use with the presently proposed control system. Applicable types include, for example, controllable pumps that give a smooth flow (such peristaltic or centrifugal pumps), or pumps that give a pulsatile flow. Where a pressure drop across the life support device (which may be a membrane oxygenator) is sufficiently low, it may not be necessary to employ a pump; the vein may be divided and whole flow in the vein directed through the life support device. Blood pressure created by a heart may be sufficient to drive blood through the life support device.

Gas concentrations are discussed herein as a percentage, which will be understood by the skilled addressee to mean molar percentage. Blood gas concentrations are typically discussed only in terms of equilibrium gas partial pressures (in kPa) or saturations. The saturations are conventionally expressed as the percentage of oxygen bound to blood haemoglobin as a proportion of the stoichiometric oxygen requirement when an oxygen is bound to every haem in the blood. Gas concentrations are referred to herein in terms of molar percentages which, at these low pressures when the ideal gas laws apply, are thought to be the same as volumetric concentrations.

In instances wherein the body requires less oxygen, the present control system preferably responds in an inverse manner to that described above. A low oxygen requirement results in little oxygen being consumed as the blood circulates the body, and consequently relatively little carbon dioxide is present in the blood. A chemoreceptor subsequently detects the low carbon dioxide concentration in the blood and reduces the circulation rate and breathing rate. This may result in a higher concentration of blood carbon dioxide until gaseous transfer occurs at a rate directly matching the rate required to support the reduced metabolic demand. It is therefore an important feature of the present invention that a reduction in oxygenation (the first gas flow rate) is enabled. Without this feature, the autonomic nervous system would reduce circulation without a consequential reduction in blood oxygen concentration or increase in blood carbon dioxide concentration. As a result, the autonomic nervous system would reduce the blood circulation rate to dangerously low levels. Indeed, in the early days of artificial blood oxygenation, several patients died because the autonomic nervous system triggered complete cessation of respiratory function. For the present control system, a very low gas transfer rate at low metabolic demand would preferably give sufficiently high carbon dioxide to avoid such extreme conditions. For this purpose, it may be desirable to provide a carbon dioxide concentration in the second gas, sufficient to trigger an increased heart rate at very low metabolic demands.

The second oxygen concentration is lower than the first oxygen concentration.

Preferably, the second gas comprises a second carbon dioxide concentration selected from the range: 0 % to 4 %.

The preference is for systems in which the first oxygen concentration is towards the upper end of the range discussed, so that if the first gas flow rate should fail (fall to zero), the individual requiring treatment will still have an oxygen supply sufficient to maintain life. In some embodiments, the second gas may also have a carbon dioxide concentration of between 0% and 4%. The upper carbon dioxide concentration value being suitable for use with a counter-current oxygenator when, otherwise, carbon dioxide partial pressure in the blood may be reduced to an excessively low value.

There is no need to measure blood gas concentration using the present invention, in order to achieve concentrations that respond to metabolic demand as they do in a normal healthy person. The present invention performs only a single measurement using the sensor, the present invention does not measure any blood gas concentration or any blood gas partial pressure. Thus, there is no setting of "target" oxygen and/or carbon dioxide concentrations as is performed in currently available devices and control systems, and there is no need for any feedback control (as employed in established systems). The natural mechanisms of the autonomic nervous system (the measurand) provides all the feedback that is needed for the present invention to operate.

The present invention is required to control a gas flow rate, the gas having a concentration of oxygen. The invention controls two gas flow rates, one with a high oxygen concentration of approximately 100% (v/v) and one with a lower concentration. The flow rates of each can be controlled to provide a gas mixture having a suitable oxygen concentration. Neither gas is preferably required to contain carbon dioxide, although carbon dioxide may possibly be present at a carbon dioxide concentration in the gas having a lower oxygen concentration.

Preferably the extracorporeal life support device is an extracorporeal membrane oxygenator.

In accordance with an aspect of the present disclosure not according to the claimed invention there is provided an extracorporeal life support device control system arranged to provide a blood flow rate through an extracorporeal life support device; the control system comprising: a sensor arranged to detect and output a measurand, wherein the measurand is characteristic of a single autonomic nervous system output defining a metabolic demand; and a controller arranged to receive the measurand, and further arranged to control, according to the measurand: a blood flow rate through an extracorporeal life support device; wherein the blood flow rate is arranged to be similar to that arising from a healthy heart at the metabolic demand.

Preferably the extracorporeal life support device controlled by the control system of the second aspect of the present disclosure not according to the claimed invention comprises a ventricular assist device (VAD) or an artificial heart.

In an example not according to the claimed invention, the measurand may be the derived from the frequency and depth of breathing. The equations corresponding to equations (1), (2) and (3) are then:

$$f_D = kv \hspace{8cm} (4)$$

$$g_1 = a + bv \hspace{7.5cm} (5)$$

$$g_2 = c + dv \hspace{7.5cm} (6)$$

Where the measurand "v" is a non-invasive measure of the ventilation rate derived, for example, from breathing rate and chest expansion. The generalizations applicable to equations (1) to (3) are equally applicable to equations (4) to (6). Thus, other algorithms are applicable including non-linear relationships between the measurand and the controlled variables. Control with one gas stream and blood flow rate, control of more than 2 gas streams, and control of flow through two or more separate sections of a life support device (such as a membrane oxygenator). Where the patient has a deficient heart, but healthy lungs, equation (4) can be applied to VAD devices and artificial hearts. "$f_D$" of equation (1) then represents the blood flow rate pumped by the VAD or artificial heart.

Preferably, the measurand is characteristic of one selected from the group: a heart rate; a blood flow rate from a blood vessel or a heart; a heart stroke volume; a pulse rate; a ventilation rate. The measurand may optionally be inferred, calculated or estimated from one or more measurands. The measurand may be a compound measurand. In an example, the measurand is heart rate estimated using a pulse rate. In another example, the measurand is heart rate estimated using the product of a pulse rate and a heart stroke volume. The measurand may also be breathing rate or depth of breathing, when the control system effectively uses the measurand to amplify the gas transfer arising in an individual's deficient lungs. This measurand is also applicable for an individual with both deficient lungs and heart. It can be used by the control system both to control the gas transfer rate and the blood circulation rate when a ventricular assist device or an artificial heart is used to pump the blood. The breathing rate/depth measurand may also be employed when the lungs are healthy but the heart is not working satisfactorily. In all these applications use is made of the observation that, in controlling the respiration rate, the autonomic nervous system normally changes ventilation rate and blood circulation rate in synchronization. When either the lungs or heart are defective, measuring the output from the other serves as a surrogate of the autonomic nervous system output and can be used as an input to control a device augmenting the function of the defective organ.

The blood flow rate through the device is preferably proportional to the heart rate and preferably arranged to be approximately a constant fraction of the blood flow through a vein from which the blood is taken. It is recognized that said fraction can, in most cases, only be made approximately constant because the blood flow through each vein does not change in exact proportion to the change in total flow from the heart.

Preferably a first gas flow rate and a second gas flow rate (two gas streams) within the extracorporeal life support device (preferably a membrane oxygenator) are controlled, one with a relatively high oxygen concentration (compared to the other) whose flow rate increases with increasing blood flow rate, and one with a relatively low oxygen concentration (relative to the other) whose flow decreases with increasing oxygen concentration.

Preferably the first gas (that with higher oxygen concentration) is approximately 100% oxygen, and the second gas (that with lower oxygen concentration) is air.

In some examples not according to the claimed invention a single gas is supplied to the extracorporeal life support device and corresponding flow rates of the single gas and blood through the life support device are controlled to produce blood gas concentrations similar to those that would be produced by healthy lungs. It will be understood to the skilled addressee that the term "similar to" is to be taken to mean "approximating" and that "healthy lungs" indicates survival is provided through continued use without intervention through any other means.

Optionally, the measurand is a blood flow rate from a blood vessel or a heart (or an approximation thereof), and wherein the controller is arranged to control, according to the measurand, the blood flow rate of an extracorporeal life support device to mimic the blood flow rate from a blood vessel or a heart. In such examples, the measurand may be derived from the heart rate on the basis of an approximately constant heart stroke volume, or from the product of the heart rate and the heart stroke volume. The control system in such examples is arranged to control, according to the measurand, the flow rate of blood through an extracorporeal life support device and to control the gas flow rate, or flow rates, through the device.

This measurand is not possible where the patient's heart is deficient, is supported by a ventricular assist device or replaced by an artificial heart. In these circumstances an alternative measurand may be the ventilation rate which may be estimated by the breathing rate or by the breathing rate and an estimate of the volume of air inhaled at each breath, for example, from the chest expansion. Where the lungs are deficient, the expansion may be limited, but the extent of the expansion still provides a measure of the output from the autonomic nervous system.

It may be difficult to measure the flow rate of blood through any specific blood vessel non-intrusively and pumping blood from that blood vessel may alter the flow rate through it. In contrast, it is very easy to measure the pulse rate non-intrusively. If we assume, for example, a constant heart stroke volume of 70 mL, it is then equally easy to estimate the blood flow rate from the heart. It is possible, using a device similar to an ECG, to measure the heart stroke volume and not too difficult to detect changes in heart stroke volume. Hence, there are relatively simple non-intrusive means of estimating the blood flow rate from the heart (essentially, the blood circulation rate). Also, it may be the blood flow rate from the heart that most closely mirrors the ventilation rate, rather than that from a blood vessel. Hence, the preferred measurand may be an estimate of the blood flow rate from the heart.

The control system does not employ a measure of any blood gas concentration or any blood gas partial pressure.

The present invention provides a control system for extracorporeal life support devices which interprets a human body's natural control outputs effected by the autonomic nervous system of an individual receiving life support. The present invention uses a sensor, or sensors, to detect a measurand, said measurand acting as a surrogate of an autonomic nervous system output. More than one sensor may be employed to obtain a single measurand. For example, the control system may measure both pulse rate and heart stroke volume and use the product thereof to estimate a single measurand, namely the blood flow rate from the heart. The measurand may, in an example, include the pulse rate of the individual receiving life support. The control system uses the measurand to control elements of an extracorporeal life support device, including flow rate of a first gas through, for example, an ECLS oxygenator. Other ECLS devices will be appreciated by the skilled addressee.

The control system of the present invention is intended to be a more elegant solution than those previously provided, and the control system does not measure a blood composition or a gas composition of an individual, and does not itself directly apply feedback control according to any predetermined set-points or thresholds of these measurands. Without these (often confounding) factors, the preferable embodiments may use the natural controls of the individual's autonomic nervous system to provide a rapid and personalised response to changes in the individual's metabolic demand, and automatically adapts to changing conditions.

The autonomic nervous system controls respiration by adjusting heart rate and ventilation rate to provide an oxygen requirement corresponding to any metabolic demand. It does so by both adjusting the blood circulation rate and the ventilation rate according to the metabolic demand. These two variables are changed by the autonomic nervous system in synchrony. A higher oxygen demand results in both a higher heart rate (pulse rate; blood circulation rate) and a deeper and more rapid breathing (higher ventilation rate). For patients with reasonably healthy hearts and deficient lungs, the autonomic nervous system increases the heart rate, but is inherently incapable of supplying the required oxygen.

The present invention employs an autonomic nervous system measurand, such as the heart rate of the individual receiving life support, as a measure of the autonomic nervous system output and adjusts the functioning of an extracorporeal life support device, such as the oxygenation rate (in an extracorporeal oxygenator) through adjustment of a first gas flow rate. This adjustment is made in synchrony with the detected change in the autonomic nervous system measurand (such as heart rate, for example). In the preferable embodiments of the system detecting heart/pulse rate and adjusting the oxygenation rate (first gas flow rate) of an extracorporeal oxygenator, the resulting ventilation rate produced by the oxygenator and the residual lung capability of the individual then preferably mimic the performance of healthy lungs. Consequently, the changes in blood composition of the individual by the system preferably results in a blood composition which matches that of a healthy person. As such, the present invention permits the autonomic nervous system of an individual to control the individual's heart and lungs in the same way as would be performed in a healthy individual. No direct measurement of blood composition or gas composition is then required.

Conventional control systems for extracorporeal life support rely on measuring blood gas composition. Measurands of blood gas composition might include equilibrium gas partial pressure, molar concentrations, and/or oxygen saturation, among others which will be apparent to the skilled addressee. Measurement of these measurands typically relies on invasive measuring devices. Such a measurement also requires a perfusionist (or a clinician) to set "desirable" targets or thresholds of such measurands, such as target oxygen and/or carbon dioxide partial pressures, in order to inform an adjustment to any life support provided. These targets or thresholds (set points) can only be set by subjective judgement and will remain the same until re-set by a clinician. In contrast, the natural "set points" inherently vary from person to person, and may vary according to the time of day, the level of exertion, and in response to changes in environmental conditions (for example, air pressure). The target or threshold set by the perfusionist may differ significantly from the value that, if left to nature, the autonomic nervous system would achieve. In a sense, the perfusionist is using conventional incomplete understanding to estimate how oxygen demand is affected by a multitude of (potentially confounding) factors, and is in essence "fighting" against the natural control system, which may contrarily lead to unstable control.

Uncertainty produced by unstable control means that a perfusionist or nurse would then need to be in 24-hour attendance to adjust the set points and to ensure that, for example, oxygenation rates of an extracorporeal oxygenator remain within sensible bounds, and that the heart rate of an individual does not become too high or too low as the autonomic nervous system seeks to impose its own control.

By delegating the control to the autonomic nervous system using the present invention, subjective judgement of set points is eliminated and the necessity of continuously monitoring gas exchange rates and compositions, through potentially invasive measurement procedures, is preferably eliminated. Furthermore, the natural "acclimatization" capability of the autonomic control system preferably eliminates the necessity of adjusting controls in response to changes in environmental conditions and changes (deterioration or improvement) in the residual lung function of the patient. The result is a much more simple and stable control system which is easier to set up and requires less-frequent monitoring. This elegance of the present solution, together with the simplicity and robustness to changes in metabolic demand, preferably reduces the extent of external human control required, and therefore makes the control system of the present invention particularly suitable for ambulatory patients. Preferable embodiments of the present system therefore permit a reduction in size of extracorporeal life support devices such that the devices are portable.

In addition to the elegance of the present solution, the control system is preferably self-adapting. A preferable advantage of integrating the present control system with the autonomic nervous system is that it inherits the natural acclimatization capability so that the characteristics of the control system do not need to mimic any particular natural lung accurately. Within a reasonably wide range of function, the autonomic nervous system will control the respiratory system (including the ECLS) so that a patient can respond to changing metabolic demands (giving mobility) and changing altitude. The acclimatization will preferably also maintain good control if a patient's residual lung capacity improves or declines.

Embodiments of the present invention may detect additional measurands relating to the functioning of an extracorporeal life support device, the additional measurands being unrelated to any indicator of the human body. The additional measurands may for example include indicators of equipment failure, ECG or pulse oximeter disconnection, among others which will be apparent to skilled addressee. Such additional measurands may be used to trigger warnings for, for example, equipment failures.

In accordance with a third aspect of the present disclosure, there is provided an extracorporeal life support device, the device comprising: a blood oxygenator comprising a blood conduit and a gas conduit; the blood conduit having a blood conduit inlet arranged to receive unprocessed blood, and a blood conduit outlet arranged to output processed blood; a gas conduit having a gas conduit inlet arranged to receive a first gas having a first oxygen concentration, and a gas conduit outlet arranged to output processed gas; a first gas supply arranged to provide the first gas from the gas conduit inlet to the gas conduit outlet at a first gas flow rate; wherein the blood conduit comprises a blood conduit lumen and the gas conduit comprises a gas conduit lumen, the blood conduit lumen and the gas conduit lumen being separated by a semi-permeable membrane disposed therebetween; and wherein the device further comprises a control system according to the first aspect of the present invention arranged to control, according to the measurand:

- the first gas flow rate.

Preferably, the device further comprises a blood pump arranged to provide blood from the blood conduit inlet to the blood conduit outlet at a blood flow rate; and wherein the control system is further arranged to the control, according to the measurand:

- the blood flow rate.

The device further comprises a second gas supply arranged to provide a second gas from the gas conduit inlet to the gas conduit outlet at a second gas flow rate; and wherein the control system is further arranged to the control, according to the measurand:

- the second gas flow rate.

In embodiments the device may comprise an oxygen concentrator arranged to adjust one or more of: oxygen concentration; and oxygen flow rate. An oxygen concentrator would be suitable for embodiments wherein the first gas is oxygen. An example oxygen concentrator is provided by Invacare (trade mark).

The device does not measure any blood gas concentration or any blood gas partial pressure.

Preferably the device is portable. In some optional embodiments, the device is wearable. The non-invasive nature of the measurands, whether from heart or lung measurements, may facilitate portability compared with the more invasive measurands of earlier control systems.

In accordance with an aspect of the present disclosure not according to the claimed invention, there is provided a method of controlling a first gas flow rate of an extracorporeal life support device having a first gas supply arranged to provide the first gas flow rate, the method comprising the steps of:

   i. connecting to an extracorporeal life support device;
   ii. using a control system having a sensor, detecting a measurand with said sensor, the measurand being characteristic of an autonomic nervous system output;

iii. calculating asuitable first gas flow rate using the measurand; and

iv. controlling the first gas supply to provide the suitable first gas flow rate.

Preferably, the method further comprises controlling a blood flow rate of the extracorporeal life support device having a blood pump arranged to provide the blood flow rate; the method further comprising the steps of:

v. calculating asuitable blood flow rate using the measurand; and

vi. controlling the blood pump to provide the suitable blood flow rate.

Preferably, the method further comprises controlling a second gas flow rate of the extracorporeal life support device having a second gas supply arranged to provide the second gas flow rate; the method further comprising the steps of:

vii. calculating asuitable second gas flow rate using the measurand; and

viii. controlling the second gas supply to provide the suitable second gas flow rate.

Examples of the disclosure will be appreciated wherein a combination of one or more of steps iii, v, and vii occur simultaneous. Further examples of the disclosure will be appreciated wherein steps iv and viii occur simultaneously. In some examples of the disclosure, the order of steps may, for example, be: step i; step ii; step iii and optionally any combination of one or more of steps v and vii, in any order or simultaneously; followed by step iv and optionally any combination of one or more of steps vi and viii, in any order or simultaneously.

Preferably, the method steps are performed by a control system in accordance with the first aspect of the present invention.

Preferably the extracorporeal life support device is in accordance with the third aspect of the present disclosure.

## Detailed Description

Specific embodiments will now be described by way of example only, and with reference to the accompanying drawings, in which:

FIG. 1 shows a schematic view of an example embodiment of an extracorporeal life support device according the third aspect of the present disclosure comprising a control system according to the first aspect of the present invention;

FIG. 2 shows a shows a schematic view of an example embodiment of an extracorporeal life support control system according to the first aspect of the present invention suitable for an ECLS system including a very low pressure drop oxygenator;

FIG. 3 shows a schematic view of an example embodiment of an extracorporeal life support control system according to the first or second aspect of the present disclosure suitable for an ECLS system applied to a patient who also has heart deficiency and needs a ventricular assist device or artificial heart;

FIG. 4 shows a schematic view of an example of a control system not according to the claimed invention suitable for a patient with healthy lungs, but with heart deficiency requiring a ventricular assist device or artificial heart; and

FIG. 5 shows a flow chart depicting an example embodiment of a method according to an aspect of the present disclosure not according to the claimed invention, using a control system according to FIG. 1.

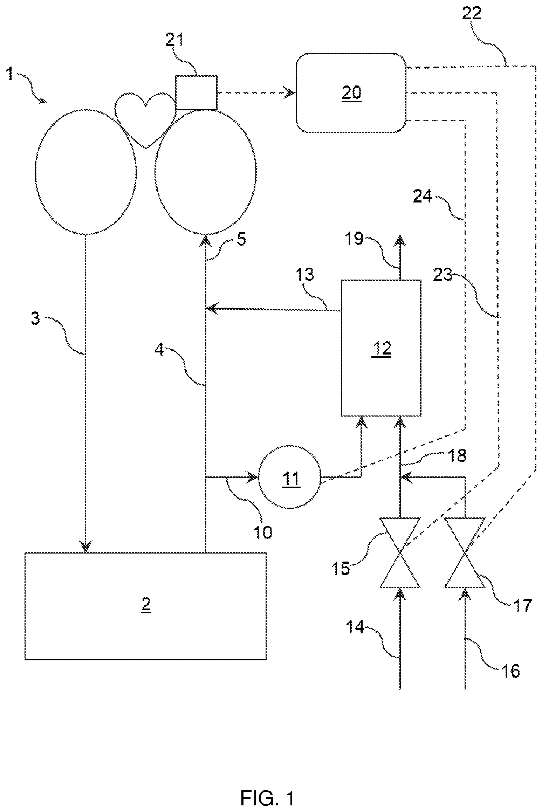

Referring to FIG. 1, a schematic diagram of an example embodiment of a control system in use according to the present invention is shown. In the embodiment of FIG. 1, a heart and lung system 1 is shown, from which oxygenated arterial blood 3 is pumped around a human body 2. In the body 2, oxygen is consumed and carbon dioxide is produced, resulting in oxygen-depleted venous blood 4, which is returned to the heart and lungs 1, where carbon dioxide is removed from the blood and the blood is re-oxygenated.

In the heart and lungs 1 shown, the blood-oxygenation function of the lungs 1 is defective, and therefore requires supplementation using an extracorporeal life support device (ECLS) to ensure adequate blood oxygenation.

In the ECLS shown in FIG. 1, a blood pump 11 is provided, the blood pump 11 being arranged to divert a portion of the venous blood 4, as stream 10, toward a mass exchanger 12. Diverted venous blood 10 is pumped by the blood pump 11 into a blood conduit (not shown) of a mass exchanger 12.

The mass exchanger 12 comprises a blood conduit and a gas conduit. The blood conduit comprises a blood conduit inlet arranged to receive the diverted venous blood 10 from the blood pump 11, and a blood conduit outlet arranged to output processed blood 13 back into the venous blood stream 4 which then returns to the heart and lung system 1.

The mass exchanger 12 also comprises a gas conduit having a gas conduit inlet arranged to receive a gas mixture 18. The gas conduit also comprises a gas conduit outlet arranged to output processed gas 19. The mass exchanger 12 comprises first gas pump 15 arranged to pump the first gas 14 into the gas conduit through the gas conduit inlet and to the gas conduit outlet at a first gas flow rate. The mass exchanger 12 also comprises a second gas pump 17 arranged to pump the second gas 16 into the gas conduit through the gas conduit inlet and to the gas conduit outlet at a second gas flow rate. The blood conduit comprises a blood conduit lumen and the gas conduit comprises a gas conduit lumen. The blood conduit lumen and the gas conduit lumen are separated by a semi-permeable membrane disposed therebetween. The semipermeable membrane forms a series of channels of the gas conduit such that a flowing gas (comprising the first gas and the second gas) may flow through the series of channels between the gas conduit inlet and the gas conduit outlet. The semi permeable membrane is arranged to permit mass transfer of oxygen and carbon dioxide between the blood conduit lumen and the gas conduit lumen such that the output processed blood 13 comprises a different gas composition to the diverted venous blood 10. ECLS is employed for patients having deficient lungs within the heart/lung system 1 who are unable to sufficiently oxygenate the blood 3. The exchanger 12 then removes carbon dioxide from the blood 10 into the gas mixture 18 within the mass exchanger 12, and oxygen is added from the gas mixture 18 to the blood 10, to produce oxygenated output blood 13 to be returned to the venous blood stream 4. The resulting oxygen-depleted gas stream 19 is discharged from the exchanger 12.

The limited capability within the heart and lung system 1 then completes the oxygenation of the blood such that the combined action of the mass exchanger 12 and the otherwise inadequate heart and lungs together produce an arterial blood stream 3 of similar composition to that of a healthy person.

The control system augments the ECLS system by controlling the rate of blood flow 10 and the flow rate and composition of the gas stream 19 in response to surrogates of the output from the autonomic nervous system. In this way, control is delegated to the autonomic nervous system.

Alternative embodiments will be appreciated in which gas stream 18 is the output from an oxygen concentrator. The oxygen concentration and flow rate from the concentrator is set by the signals 22 and 23 from the controller.

The control system of the present invention comprises a sensor 21, in digital communication with a control unit 20, which in-turn is in digital communication with the blood pump 11, the first gas pump 15, and the second gas pump 17. The blood pump 11 is linked to the controller by a first digital connection 24. The first gas pump 15 is linked to the controller by a second digital connection 23, and the second gas pump 17 is linked to the controller by a third digital connection 22.

The first, second and third digital connections 22, 23 and 24 in the embodiment shown are wireless connections. Embodiments will be appreciated wherein the connections are wired or wireless, or any combination of wired and wireless. Embodiments will also be appreciated wherein the connections are wired or wireless analogue signals, and may be pneumatic or hydraulic, or optical.

The sensor 21 is arranged to detect a pulse rate of the heart 2. Embodiments will be appreciated wherein the sensor 21 is arranged to sense any surrogate of the output from the autonomic nervous system which, in a healthy person, controls the rate at which blood is pumped around the body and the breathing rate and depth. In a healthy person, there is a relationship between the rate at which the blood is pumped around the body and the ventilation rate (the quantity of air that enters and leaves the lungs in any defined period of time), provided that the person is in sinus rhythm. The ventilation rate and blood circulation rate together determine the rate of oxygen and carbon dioxide transfer in the lungs.

The present control system, in the embodiment shown in FIG. 1, takes a measure of the blood flow rate using the sensor 21, and the measurement is transmitted to the control unit 20. The control unit subsequently provides a signal 24 to the blood pump 11, wherein following receipt of the signal, the blood flow rate from the blood pump 11 through the blood conduit of the mass exchanger 12 is altered by the blood pump 11 in proportion to the blood flow rate measured from the heart and lungs 1. The rationale for this control is that the blood flow rate through a vein is roughly proportional to the flow rate from the heart and lungs. The control using the present system ensures that an approximately constant proportion of the blood flow through a vein is taken through the mass exchanger 12.

The measure of the blood flow rate taken by the sensor 21 is further used by the controller 20 to determine and provide a signal 23 to the first gas pump 15 and a signal 22 to the second gas pump 17. Signals 23 and 22, upon receipt by their respective gas pumps 15, 17 adjust the gas flow rates of the respective gases 14, 16 through the gas pumps 15, 17. The altered gas flow rates through the gas pumps 15, 17 determines the composition and flow rate of the mixed gas stream 18 entering the gas conduit of the mass exchanger 12.

A specific embodiment of the control algorithm employs equations (1), (2) and (3) described herein, where the measurand is:

$f$    is the blood flow rate from the heart as estimated by sensor 21; and the controlled variables are:

$f_D$    is the blood flow rate of streams 10 and 13 as set by pump 11;

$g_1$    is the flow rate of gas stream 14 as set by control valve or pump 15;

$g_2$ is the flow rate of gas stream 16 as set by control valve or pump 17;

Other algorithms are possible in which there are non-linear relationships between the measured variable "f" and the controlled variables set by pumps or control valves.

In the specific embodiment shown, gas stream 14 is oxygen and gas stream 16 is air. Other embodiments are possible in which gas stream 14 is relatively oxygen-rich and gas stream 14 has a comparatively lower oxygen concentration. Gas stream 14 may also contain a small concentration of carbon dioxide, such as that discussed herein.

In a further specific embodiment, the control algorithm employs equations (4), (5) and (6), where "v" is the ventilation rate as estimated by the sensor 21. For a person with healthy lungs, it may be the healthy ventilation rate. For a person with deficient lung function, it may be a fraction of the healthy ventilation rate. As for sensed variable "f", alternative algorithms are possible, and embodiments with gas streams of alternative composition are possible. The embodiment shown represents a simple feed-forward control as in the above equations. There is no feedback in the embodiment shown - the feedback is left to the autonomic nervous system.

In conventional control systems such as those found in the prior art, blood oxygen partial pressure or concentration, x, is typically measured. A perfusionist sets a target "healthy" concentration, say $x_T$. The control is then along the following lines:

$$dg_1/dt = K(x_T - x) \qquad\qquad (7)$$

The equation has the effect of increasing the oxygen flow rate if x is less than the target concentration and decreasing if it is higher. The system settles when the oxygen flow rate matches the target rate. Similar controls are applied to achieve target carbon dioxide concentrations.

The first gas flow rate and the second gas flow rate are adjusted by the controller to alter the oxygen and carbon dioxide concentrations in the mixed gas stream 18 flowing into the mass exchanger. This adjustment is in accordance with the measurand (the venous blood flow rate or ventilation rate detected by the sensor 21). The adjustments are tailored to produce blood gas concentrations in arterial blood stream 3 from the heart and lungs 1 which mimic those arising in a person with healthy lungs.

In the embodiment shown, the first gas 14 is oxygen, the second gas 16 is air, and the flow of blood through the blood conduit and the flow of gas through the gas conduit occurs in a co-current fashion. The benefit of a co-current mass exchanger is that outlet blood gas partial pressures (or concentrations) are nearly in equilibrium with the outlet gas stream 19. When the gas flow rate through the mass exchanger 12 is very high, the concentration of carbon dioxide in the outlet stream 19 is very low because the carbon dioxide concentration transferred from the blood is a small proportion of the gas flow rate through the device 12. Conversely, when the gas flow rate is very low, the carbon dioxide concentration in output stream 19 is very high because the carbon dioxide transferred in the exchanger is a significant proportion of the gas flow rate through the device. In this way, the carbon dioxide concentration in gas stream 19 (and hence the partial pressure and concentration in blood stream 13) can be controlled by adjusting the total gas flow rate through the exchanger 12.

The oxygen transfer rate (and hence the oxygen partial pressure and concentration in blood stream 13) is controlled by controlling the oxygen concentration in stream 18. The complete control system then operates as follows. At highest metabolic demand (as signalled by maximum blood flow rate detected by sensor 21) oxygen stream 14 is at a maximum flow rate and air stream 16 is at zero flow rate. At minimum metabolic demand the oxygen 14 flow rate is zero and the air 16 flow rate is slightly higher, thus the total gas flow rate 18 is low and the major portion is made up of air 16 (which has a lower oxygen concentration). The resulting system closely mimics healthy lungs by implementing a linear relationship between oxygen flow rate 14 and blood circulation rate (detected by sensor 21), and by implementing a linear relationship between air flow rate 16 and blood circulation rate.

Alternative embodiments are possible in which the mass exchanger may be cross-current or counter-current. For a cross-current mass exchanger, a wider range of total gas flow rate 18 into the exchanger is required because outlet blood gas partial pressures have a more complex relationship with outlet gas composition which is a mixture between gas that exits in contact with the inlet blood stream and some exits in contact with the blood outlet stream. For a counter-current mass exchanger, the outlet blood stream is in contact with the inlet gas stream. Hence, in this case, stream 16 will need to contain carbon dioxide to give a sufficiently high partial pressure (and concentration) of carbon dioxide in outlet blood stream 13.

In the embodiment shown, the flowing gas may flow freely through the series of cylindrical channels of the gas conduit formed by the semipermeable membrane. Embodiments will be appreciated wherein access to, or flow rate through, one or more of the series of channels may be controllable by the control system. As such, the control system may, according to the measurand, increase or reduce access of the flowing gas to the one or more of the series of channels in order to increase or reduce the effective area of the mass exchanger. It will be appreciated that, in substantially the same manner in an embodiment wherein the series of channels are instead comprised within the blood conduit, the control system may, according to the measurand, increase or reduce access of the blood to the one or more of the series of channels in order to increase or reduce the effective area of the mass exchanger.

Embodiments will be appreciated wherein a flat-sheet mass-exchanger is provided, wherein the gas conduit or the blood conduit may comprise a series of parallel channels.

In further embodiments, the sensor may sense pulse rate instead of blood flow rate. Blood flow rate is the product of pulse rate and stroke volume and for modest exertion, stroke volume does not change greatly with metabolic demand. Hence, pulse rate can stand in as a surrogate for blood flow rate and for output from the autonomic nervous system. It would be expected that the ability of the autonomic nervous system to acclimatize would adapt to such a control.

Various non-invasive methods are available for capturing the measurand, which for blood flow rate is made up from the product of pulse rate and heart stroke volume. For example, the pulse rate may be measured with an ECG and the stroke volume estimated from methods based on measuring pulse wave velocity. Alternatively, a fixed value of stroke volume may be employed which may be related to the size of the patient; a typical value would be 70 ml. Ventilation rate is made up from the product of breathing rate and volume of each breath; estimates of both are available without using a face mask.

The blood pump 11 is a positive displacement pump, so that the flow rate can be directly controlled. Where a pump that is not positive displacement (such as a centrifugal pump) is employed, the blood flow rate can be set as a set-point on a feedback control that measures flow rate.

Alternative embodiments will be appreciated wherein the first gas is not pure oxygen, but comprises oxygen at a suitable concentration or partial pressure. Embodiments will be appreciated wherein the second gas is not air, but simply comprises oxygen at a lower concentration or partial pressure compared to the first gas. The first gas and the second gas may be used having differing oxygen concentrations and possibly carbon dioxide concentrations to those discussed.

The first gas supply and the second gas supply in the embodiment shown are provided through metering pumps 15, 17. The first gas supply and the second gas supply in alternative embodiments may comprise flow metering valves from high pressure (for example, bottled) gas supplies. One of the gas supplies may be from a portable oxygen concentrator with controllable flow rate and composition. Alternatively, there may be only one gas supply delivered from a controllable portable concentrator.

In some cases, the controller can be calibrated by determining two points on a calibration curve. The first point corresponds to the maximum metabolic rate that can be supported with the maximum oxygen concentration available and adjusting the relevant gas flow rate to give a stable carbon dioxide concentration. The second point corresponds to resting conditions, when the lowest oxygen concentration to support rest is established together with a relevant gas flow rate to give a stable carbon dioxide concentration. The controller is then programmed to interpolate smoothly between these two points (for example, with linear interpolation). Note that the resting rate must not be the lowest possible oxygenation rate and highest carbon dioxide concentration capable of being set by the control unit. Conditions can arise at which the patient's metabolic rate falls below the resting rate. Under such conditions, the control must supply a sufficiently low gas flow rate, or high carbon dioxide concentration, to prompt the autonomic nervous system to increase the respiration rate. If this condition is not met, the respiration rate may continue to fall without limit. The calibration may be performed directly on a patient, or may be performed on a mathematical model of the patient's respiratory system. Undertaking the calibration on a mathematical model may minimize the stress on a patient already suffering with breathing difficulties.

The novel control system presented here is equally applicable to alternative extracorporeal life support systems, and indeed to control of ventricular assist devices and artificial hearts.

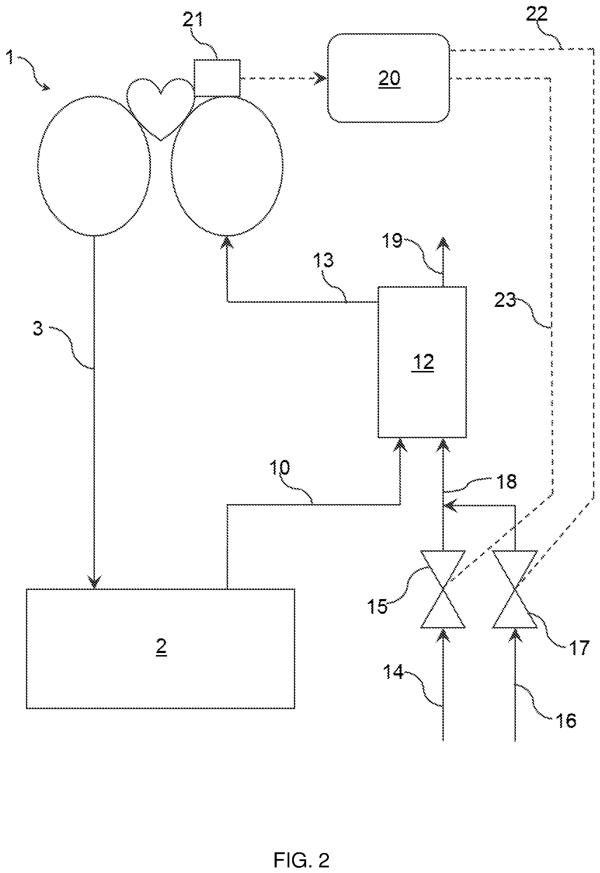

FIG. 2 shows an ECLS system comprising features as described and shown in FIG. 1, and equivalent numbering is therefore used. The ECLS system of FIG. 2 includes a very low pressure drop for the blood stream through mass exchanger 12. With such a low pressure drop, a blood pump is not necessary and the whole flow through a vein can be directed through the mass exchanger 12 (in this case a membrane oxygenator). In this case, the control unit 20 needs only control the flow rate and composition of mixed stream 18 into the mass exchanger. It does so by controlling the flow rates of gases 14 and 15 in the identical manner to that described for FIG. 1. The criterion for a "very low" pressure drop across the exchanger depends on the vein from which the blood is taken and the pressure differential available in the vein without having an adverse impact on the blood flow.

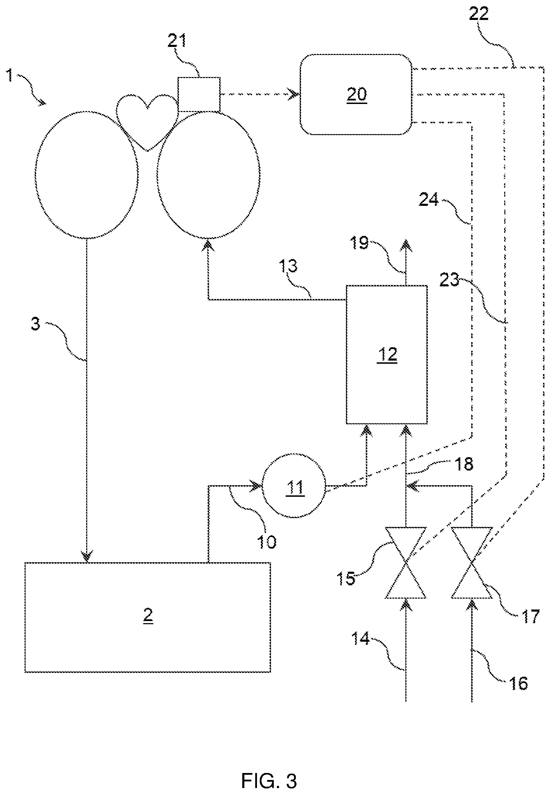

FIG. 3 shows an ECLS system employed for a patient who also has impaired heart function. The system comprises features as described and shown in FIG. 1, and equivalent numbering is therefore used. For patients also having impaired heart function, the ECLS system now takes the whole flow in the blood vessel. The blood pump 11 in this case is a ventricular assist device, but can be any number of suitable blood pumps as described herein. The measurand from sensor 21, the surrogate for the output from the autonomic nervous system, is an estimate of the ventilation rate. The control system 20 controls both the blood flow 10 through blood vessel on input to the exchanger 12, the blood flow 13 on output from the exchanger 12 and the gas transfer rate in the exchanger 12. Apart from the higher pressure required from the pump 11, the operation is identical to that described for FIG. 1. Where the patient needs an artificial heart, its function will be integrated with the heart/lung system and it will be located between blood flow 13 and blood flow 10. In these applications, the life support device 12 (in this case a membrane oxygenator) may be located before pump 11 or after the heart/lung system. In the latter case, the oxygenator completes the limited gas transfer in the deficient lungs. As is the case for FIG. 1 and FIG. 2, the control is delegated to the autonomic nervous system; when the blood is insufficiently oxygenated for the level of activity (metabolic demand), the patient breathes harder. The sensor 21 detects the increased breathing effort and increases blood circulation and gas transfer rate until a sufficient rate is established. Similarly, where the blood is oxygenated at a rate higher than required, the breathing effort reduces and the controller reduces blood circulation and gas transfer.

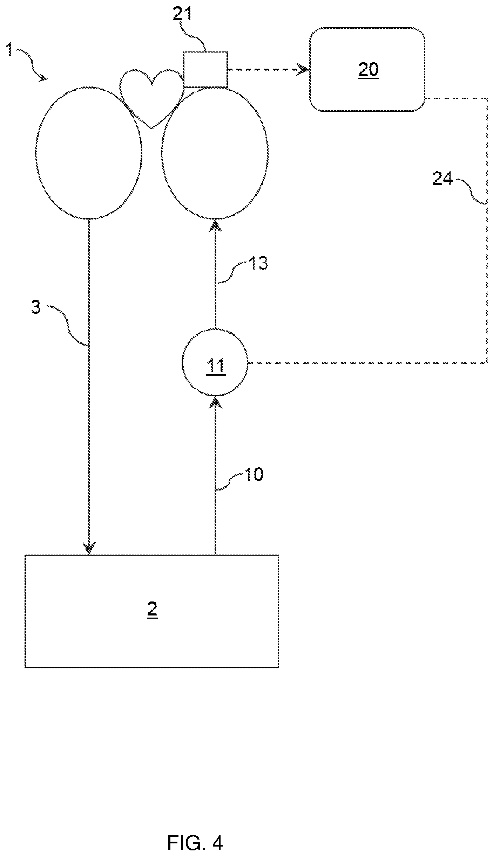

FIG. 4 shows the control system adapted for use with a patient with healthy lungs but defective heart. In this case, the mass exchanger 12 of FIG. 3 is omitted and control unit 20 controls only the pump 11, which may be a ventricular assist device or an artificial heart. The ventilation rate estimated by the sensor 21 enables the pumping rate to be adjusted to meet the required metabolic demand. This control integrated with the autonomic nervous system is simpler and more responsive than previous control systems. The case for this integrated control is exactly similar to that for ECLS systems.

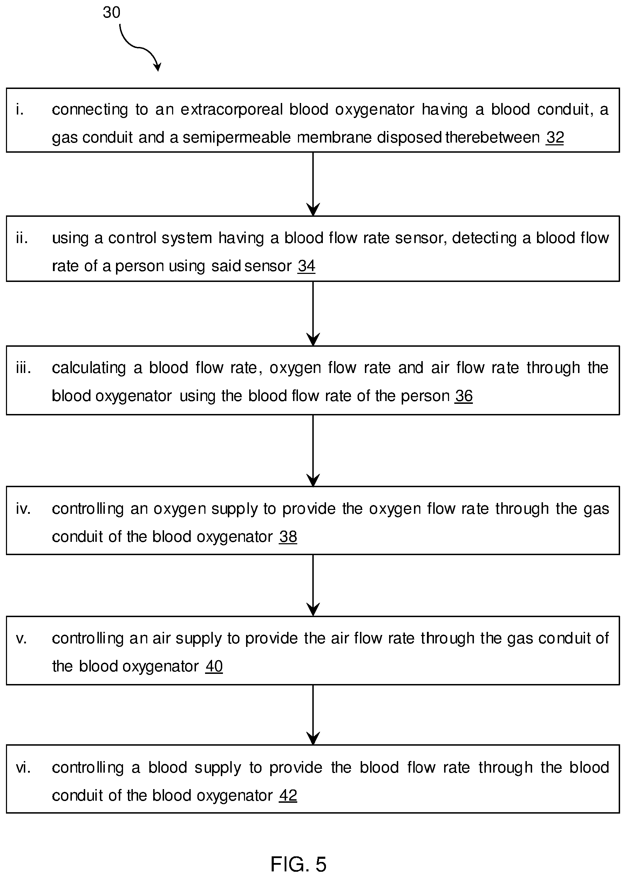

Referring to FIG. 5, a flow chart of an example embodiment 30 of the method of an aspect of the present disclosure not according to the claimed invention is shown, the method 30 comprising the steps of:

   i. connecting to an extracorporeal blood oxygenator having a blood conduit, a gas conduit and a semipermeable membrane disposed therebetween 32;
   ii. using a control system having a blood flow rate sensor, detecting a blood flow rate of a person using said sensor 34;
   iii. calculating an oxygen flow rate using the blood flow rate 36; and
   iv. controlling an oxygen supply to provide the oxygen flow rate through the gas conduit 38;
   v. calculating a blood flow rate using the measurand 40; and
   vi. controlling a blood pump to provide the blood flow rate 42;
   vii. calculating a second gas flow rate using the measurand 44; and
   viii. controlling the second gas supply to provide the second gas flow rate 46.

The control system used in the method of FIG. 5 is a control system in accordance with that described for FIG. 1, and according to the first aspect of the invention. A similar method will be appreciated using a control system according to that described for FIG. 2, FIG. 3 or FIG. 4.

It will be appreciated that the above described embodiments are given by way of example only and that various modifications thereto may be made. The invention is defined in the appended claims.

Claims

1. An extracorporeal life support device control system arranged to provide a gas flow rate through an extracorporeal life support device (12); the control system comprising:

   a sensor (21) arranged to detect and output a measurand, wherein the measurand is characteristic of a single autonomic nervous system output defining a metabolic demand;
   and
   a controller (20) arranged to receive the measurand, and further arranged to control, according to the measurand:

      - a first gas flow rate of a first gas (14) through an extracorporeal life support device, the first gas having a first oxygen concentration of approximately 100% (v/v);
      and
      - a second gas flow rate of a second gas (16) through the extracorporeal life support device, the second gas having a second non-zero oxygen concentration;

   wherein the second oxygen concentration is lower than the first oxygen concentration;
   wherein the control system does not measure any blood gas concentration or any blood gas partial pressure; and
   wherein the first gas flow rate and the second gas flow rate is arranged to provide blood gas concentrations similar to those arising from healthy lungs at the metabolic demand

2. An extracorporeal life support device control system as claimed in claim 1, wherein the controller is further arranged to control, according to the measurand:

      - a blood flow rate through the extracorporeal life support device.

3. An extracorporeal life support device control system as claimed in claim 2, wherein the controller is arranged to control a blood pump (11) of the extracorporeal life support device to control the blood flow rate.

4. An extracorporeal life support device control system as claimed in claim 3, wherein the blood pump is one selected from the group: a peristaltic pump; a continuous flow pump; a centrifugal pump; a pulsatile pump; any future blood pump design.

5. An extracorporeal life support device control system as claimed in any one of the preceding claims, wherein the second oxygen concentration is selected from the range: 0 % to 25%.

6. An extracorporeal life support device control system as claimed in any one of the preceding claims, wherein the second gas comprises a carbon dioxide concentration selected from the range: 0 % to 4 %.

7. An extracorporeal life support device control system as claimed in any one of the preceding claims, wherein the extracorporeal life support device is an extracorporeal membrane oxygenator.

8. An extracorporeal life support device control system as claimed in any one of the preceding claims, wherein the measurand is characteristic of one selected from the group: a pulse rate; a blood flow rate from a heart; a ventilation rate.


**Patentansprüche**

1. Ein Steuersystem für eine extrakorporale Lebensunterstützungsvorrichtung, das eingerichtet ist, um eine Gasflussrate durch eine extrakorporale Lebensunterstützungsvorrichtung (12) bereitzustellen; wobei das Steuersystem Folgendes beinhaltet:

   einen Sensor (21), der eingerichtet ist, um eine Messgröße zu erfassen und auszugeben, wobei die Messgröße für eine einzelne Ausgabe des autonomen Nervensystems charakteristisch ist, die einen metabolischen Bedarf definiert; und
   eine Steuerung (20), die eingerichtet ist, um die Messgröße zu empfangen, und die ferner eingerichtet ist, um gemäß der Messgröße Folgendes zu steuern:

   - eine erste Gasflussrate eines ersten Gases (14) durch eine extrakorporale Lebensunterstützungsvorrichtung, wobei das erste Gas eine erste Sauerstoffkonzentration von ungefähr 100 % (v/v) aufweist; und
   - eine zweite Gasflussrate eines zweiten Gases (16) durch die extrakorporale Lebensunterstützungsvorrichtung, wobei das zweite Gas eine zweite Sauerstoffkonzentration ungleich null aufweist;

   wobei die zweite Sauerstoffkonzentration niedriger als die erste Sauerstoffkonzentration ist;
   wobei das Steuersystem keine Blutgaskonzentration und keinen Blutgaspartialdruck misst; und
   wobei die erste Gasflussrate und die zweite Gasflussrate eingerichtet sind, um Blutgaskonzentrationen bereitzustellen, die denjenigen ähnlich sind, die von gesunden Lungen beim metabolischen Bedarf entstehen.

2. Steuersystem für eine extrakorporale Lebensunterstützungsvorrichtung gemäß Anspruch 1, wobei die Steuerung ferner eingerichtet ist, um gemäß der Messgröße Folgendes zu steuern:

   - eine Blutflussrate durch die extrakorporale Lebensunterstützungsvorrichtung.

3. Steuersystem für eine extrakorporale Lebensunterstützungsvorrichtung gemäß Anspruch 2, wobei die Steuerung eingerichtet ist, um eine Blutpumpe (11) der extrakorporalen Lebensunterstützungsvorrichtung zu steuern, um die Blutflussrate zu steuern.

4. Steuersystem für eine extrakorporale Lebensunterstützungsvorrichtung gemäß Anspruch 3, wobei die Blutpumpe eine ist, die aus der folgenden Gruppe ausgewählt ist: eine peristaltische Pumpe; eine Durchflusspumpe; eine Zentrifugalpumpe; eine pulsierende Pumpe; jede zukünftige Blutpumpenkonstruktion.

5. Steuersystem für eine extrakorporale Lebensunterstützungsvorrichtung gemäß einem der vorhergehenden Ansprüche, wobei die zweite Sauerstoffkonzentration aus dem folgenden Bereich ausgewählt ist: 0 % bis 25 %.

**6.** Steuersystem für eine extrakorporale Lebensunterstützungsvorrichtung gemäß einem der vorhergehenden Ansprüche, wobei das zweite Gas eine Kohlendioxidkonzentration beinhaltet, die aus dem folgenden Bereich ausgewählt ist: 0 % bis 4 %.

**7.** Steuersystem für eine extrakorporale Lebensunterstützungsvorrichtung gemäß einem der vorhergehenden Ansprüche, wobei die extrakorporale Lebensunterstützungsvorrichtung ein extrakorporaler Membranoxygenator ist.

**8.** Steuersystem für eine extrakorporale Lebensunterstützungsvorrichtung gemäß einem der vorhergehenden Ansprüche, wobei die Messgröße für eines charakteristisch ist, das aus der folgenden Gruppe ausgewählt ist: eine Pulsrate; eine Blutflussrate von einem Herzen; eine Atemfrequenz.


**Revendications**

**1.** Un système de commande de dispositif de support de vie extracorporel agencé pour fournir un débit de gaz à travers un dispositif de support de vie extracorporel (12) ; le système de commande comprenant :

un capteur (21) agencé pour détecter et délivrer en sortie un mesurande, dans lequel le mesurande est caractéristique d'une sortie de système nerveux autonome unique définissant une exigence métabolique ; et
un organe de commande (20) agencé pour recevoir le mesurande, et agencé en outre pour commander, selon le mesurande :

- un premier débit de gaz d'un premier gaz (14) à travers un dispositif de support de vie extracorporel, le premier gaz ayant une première concentration en oxygène d'environ 100 % (v/v) ; et
- un deuxième débit de gaz d'un deuxième gaz (16) à travers le dispositif de support de vie extracorporel, le deuxième gaz ayant une deuxième concentration en oxygène non nulle ;

dans lequel la deuxième concentration en oxygène est inférieure à la première concentration en oxygène ;
dans lequel le système de commande ne mesure aucune concentration de gaz sanguin ou pression partielle de gaz sanguin ; et
dans lequel le premier débit de gaz et le deuxième débit de gaz sont agencés pour fournir des concentrations de gaz sanguin similaires à celles provenant de poumons sains à l'exigence métabolique.

**2.** Le système de commande de dispositif de support de vie extracorporel tel que revendiqué dans la revendication 1, dans lequel l'organe de commande est en outre agencé pour commander, selon le mesurande :

- un débit de sang à travers le dispositif de support de vie extracorporel.

**3.** Le système de commande de dispositif de support de vie extracorporel tel que revendiqué dans la revendication 2, dans lequel l'organe de commande est agencé pour commander une pompe à sang (11) du dispositif de support de vie extracorporel pour commander le débit de sang.

**4.** Le système de commande de dispositif de support de vie extracorporel tel que revendiqué dans la revendication 3, dans lequel la pompe à sang est une choisie dans le groupe : une pompe péristaltique ; une pompe à écoulement continu ; une pompe centrifuge ; une pompe pulsatile ; toute conception de pompe à sang future.

**5.** Le système de commande de dispositif de support de vie extracorporel tel que revendiqué dans l'une quelconque des revendications précédentes, dans lequel la deuxième concentration en oxygène est choisie dans la plage : 0 % à 25 %.

**6.** Le système de commande de dispositif de support de vie extracorporel tel que revendiqué dans l'une quelconque des revendications précédentes, dans lequel le deuxième gaz comprend une concentration en dioxyde de carbone choisie dans la plage : 0 % à 4 %.

**7.** Le système de commande de dispositif de support de vie extracorporel tel que revendiqué dans l'une quelconque des revendications précédentes, dans lequel le dispositif de support de vie extracorporel est un oxygénateur à membrane extracorporel.

8. Le système de commande de dispositif de support de vie extracorporel tel que revendiqué dans l'une quelconque des revendications précédentes, dans lequel le mesurande est caractéristique d'un choisi dans le groupe : une fréquence de pouls ; un débit de sang provenant d'un cœur ; un débit ventilatoire.

FIG. 1

FIG. 2

FIG. 3

FIG. 4

30

| i. | connecting to an extracorporeal blood oxygenator having a blood conduit, a gas conduit and a semipermeable membrane disposed therebetween 32 |

| ii. | using a control system having a blood flow rate sensor, detecting a blood flow rate of a person using said sensor 34 |

| iii. | calculating a blood flow rate, oxygen flow rate and air flow rate through the blood oxygenator using the blood flow rate of the person 36 |

| iv. | controlling an oxygen supply to provide the oxygen flow rate through the gas conduit of the blood oxygenator 38 |

| v. | controlling an air supply to provide the air flow rate through the gas conduit of the blood oxygenator 40 |

| vi. | controlling a blood supply to provide the blood flow rate through the blood conduit of the blood oxygenator 42 |

FIG. 5

**EP 3 972 664 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2004255 A